Europäisches Patentamt

(19) European Patent Office

Office européen des brevets

(11) Numéro de publication : **0 313 435 B1**

(12) **FASCICULE DE BREVET EUROPEEN**

(45) Date de publication du fascicule du brevet :
05.06.91 Bulletin 91/23

(51) Int. Cl.⁵ : **G01R 33/44, G01N 33/44**

(21) Numéro de dépôt : **88402550.3**

(22) Date de dépôt : **10.10.88**

(54) **Procédé et dispositif pour la caractérisation par son taux de gel d'un échantillon de matière plastique réticulée fabriqué en continu.**

(30) Priorité : **12.10.87 FR 8714045**

(43) Date de publication de la demande :
**26.04.89 Bulletin 89/17**

(45) Mention de la délivrance du brevet :
**05.06.91 Bulletin 91/23**

(84) Etats contractants désignés :
**BE DE ES GB IT NL**

(56) Documents cités :
**WO-A-84/00066**
**JOURNAL OF MACROMOLECULAR SCIENCE - Reviews in macromolecular chemistry and physics, vol. C-25, no. 4, 1985, pages 481-489, Marcel Dekker, Inc.; D.J.P. HARRISON et al.: "Techniques for the analysis of crosslinked polymers"**
**POLYMER JOURNAL, vol. 18, no. 11, 1986, pages 859-864, Tokyo, JP; T. KUROTU: "Pulsed NMR investigation on the polymerization of methyl methacrylate"**
**MACROMOLECULES, vol. 14, 1981, pages 284-288, American Chemical Society, New York, US; W.T. FORD et al.: "Carbon-13 nuclear magnetic resonance relaxation in cross-linked polystyrene gels"**

(56) Documents cités :
**JOURNAL OF APPLIED PHYSICS, vol. 60, no. 4, 15 août 1986, pages 1306-1309, American Institute of Physics, Woodbury, New York, US; H. TANAKA et al.: "Real-time pulsed nuclear magnetic resonance measurement system for the study of nonequilibrium phenomena in polymers"**
**COLLOID & POLYMER SCIENCE, vol. 264, no. 6, 1986, pages 482-487, Darmstadt, DE; D. GESCHKE et al.: "Dynamic processes in polymeric networks as studied by NMR relaxation methods"**

(73) Titulaire : **INSTITUT FRANCAIS DU PETROLE**
**4, Avenue de Bois-Préau**
**F-92502 Rueil-Malmaison (FR)**

(72) Inventeur : **Cohen Addad, Jean-Pierre**
**25 bis, cours Berriat**
**F-38000 Grenoble (FR)**
Inventeur : **Schmitt, Carlo**
**16, rue Alphonse Allais**
**F-38400 Saint Martin D'Heres (FR)**
Inventeur : **Boscher, Yves**
**27, rue Victor Hugo**
**F-92400 Courbevoie (FR)**
Inventeur : **Roussel, Jean-Claude**
**3, Allée de la Cascade**
**F-92500 Rueil Malmaison (FR)**
Inventeur : **Jarrin, Jacques**
**31, rue du Gué**
**F-92500 Rueil Malmaison (FR)**

EP 0 313 435 B1

EP 0 313 435 B1

## Description

Le domaine technique considéré est celui du contrôle en cours de fabrication du degré de réticulation d'objets en matière plastique réticulée ou vulcanisée. Par contrôle en cours de fabrication on entend soit un contrôle en continu sur le produit en défilement en cours de fabrication soit un contrôle non destructif en discontinu par prélèvement d'échantillon avec une durée d'analyse suffisamment courte pour pouvoir corriger rapidement les conditions de fabrication.

Par matière plastique réticulée on entend soit des polymères thermoplastiques réticulés (par péroxyde, par irradiation ou par silane) soit des élastomères vulcanisés (par peroxyde, ou soufre...) soit encore des résines thermodurcissables telles que les résines epoxy, polyuréthane, polyester, etc... Ces matières plastiques peuvent être formulées avec tous les additifs et charges généralement utilisées (antioxydant, plastifiant, anti UV, charges de renfort diverses). La réticulation ou vulcanisation ou gélification traduit le fait qu'une partie plus ou moins importante de la matière plastique est devenue insoluble dans un bon solvant du même matériau non réticulé. Cette réticulation se fait par réaction des chaines de polymère ou de prépolymère les unes sur les autres et permet en général d'améliorer très sensiblement les propriétés du matériau (élasticité des élastomères, tenue thermique des polyoléfines, propriétés mécaniques des thermodurcissables, etc...). De plus amples informations sur l'intérêt que présentent les différents modes de réticulation des matériaux plastiques seront trouvés dans "Encyclopedia of polymer Science and engineering" edited by J. Wiley and sons, 2nd edition, vol. 4, p. 349 à 449.

La fabrication des objets en matières plastiques réticulés se fait généralement en 2 étapes :
– Mise en forme des objets par extrusion injection ou calandrage sous forme de tubes, de profilés ou de gaines déposés ou non sur un support dans le cas des thermoplastiques ou des élastomères. Elle se fait par pultrusion, par enroulement filamentaire, par coulée, par moulage, par injection dans le cas des résines thermodurcissables.
– La réticulation des objets est faite après la phase de mise en forme par passage dans une unité de réticulation qui a le plus souvent pour fonction d'élever la température de l'objet en défilement pour déclencher le processus de réticulation. Ce type d'unité est généralement un four ou un bain chauffant mais peut aussi être un bain humide ou une source de rayonnement ionisant.

Le contrôle de la fabrication de ce type de produit pose souvent un problème car la qualité des produits obtenus dépend de la qualité de la mise en forme (contrôle dimensionnel) et pour une grande part du degré et de l'homogénéïté de la réticulation au sein de la matière employée.

En ce qui concerne le contrôle dimensionnel des produits, de nombreuses techniques sont disponibles du type palpeurs de surface, mesure d'épaisseur par ultrasons ou IR, mesure des tailles par masquage de faisceaux lumineux, présence d'hétérogénéïté dans l'épaisseur du matériau par rayonnement X, etc...

L'utilisation de la résonance magnétique nucléaire du proton (RMN) est décrite dans le document JMS-REV. MACROMOL. CHEM. PHYS., vol. C-25, no. 4, 1985, pages 481-489, Marcel Dekker, inc. ; D.J.P. HARRISON et al. : "Techniques for the analysis of crosslinked polymers", pour l'étude de la réticulation de polymères vulcanisés par rayonnement, comme le polyéthylène. La densité de réticulation augmente d'autant plus qu'une fraction des protons, se relaxant rapidement après excitation, est importante.

L'art antérieur est par ailleurs illustré dans les documents suivants :
POLYMER JOURNAL, vol. 18, no. 11, 1986, pages 859-864, Tokyo, JP ; T. KUROTU : "Pulsed NMR investigation on the polymerization of methyl methacrylate",
MACROMOLECULES, vol. 14, 1981, pages 284-288, American Chemical Society, New York, US ; W.T. FORD et al. : "Carbon-13 nuclear magnetic resonance relaxation in cross-linked polystyrene gels",
JOURNAL OF APPLIED PHYSICS, vol. 60, no. 4, 15 août 1986, pages 1306-1309, American Institute of Physics, New York, US ; H. TANAKA et al. : "Real-time pulsed nuclear magnetic resonance measurement system for the study of nonequilibrium phenomena in polymers",
COLLOID & POLYMER SCIENCE, vol. 264, no. 6, 1986, pages 482-487 ; D. GESCHKE et al. : "Dynamic processes in polymeric networks as studied by NMR relaxation methods".

Aucun de ces documents ne décrit ni ne suggère l'utilisation en continu d'une technique de RMN dans un procédé de fabrication d'un produit polymérique.

Le contrôle de la qualité de la réticulation est très délicat et à fortiori sur la base d'analyses très rapprochées dans le temps et à ce jour on ne dispose souvent en ligne que de techniques indirectes et grossières tel que l'aspect de surface du produit ou la mesure de sa dureté de surface pour estimer en ligne le degré de réticulation. En fait ce type de contrôle est le plus souvent fait par prélèvement d'échantillons représentatifs des fabrications et caractérisation de ces échantillons par des techniques conventionnelles telles que la détermination du degré de réticulation par extraction du solvant, la mesure de la transition vitreuse par analyse thermique différentielle (DSC), la spectroscopie IR ou la caractérisation mécanique des échantillons.

2

Ces méthodes donnent généralement des informations fiables et représentatives du degré d'avancement de la réaction de réticulation dans le matériau mais présentent l'inconvénient de n'être pas applicable en routine sur la totalité de la fabrication et de nécessiter parfois des temps d'analyse assez longs.

C'est en particulier le cas pour la détermination des taux de gel. Cette méthode fait l'objet d'une norme ASTM D2756. Dans le cas des polyoléfines, l'essai consiste à placer un échantillon dans une nacelle, elle même placée dans un extracteur de type SOXLHET fonctionnant au reflux de xylène à 140°C environ. Après 8 h d'extraction, la nacelle est retirée du ballon, séchée à l'étuve sous vide à 80°C pendant environ 12 h puis l'échantillon est pesé jusqu'à poids constant. Le taux de gel est exprimé comme étant la quantité de matière non extractible rapportée à la quantité totale de matière :

$$\% \ gel = \frac{Pa}{Po \ initial} \quad avec \quad \begin{array}{l} Pa \ : \ poids \ matière \ insoluble \\ Po \ : \ poids \ initial \ échantillon \end{array}$$

Cette méthode nécessite au moins 24 heures avant d'obtenir un résultat et ne peut donc être utilisée pour contrôler des produits en cours de fabrication. En effet le temps de retour de résultats est trop long pour permettre de corriger immédiatement la mauvaise qualité du produit fabriqué par la modification des paramètres de fonctionnement de l'unité de fabrication (vitesse, température, temps de séjour...). Des rebuts de fabrication sont ainsi générés qui induisent des surcouts de fabrication.

La nouvelle méthode proposée permet de remédier à ces difficultés par une mesure rapide, non destructive et fiable du taux de gel d'un échantillon en matière plastique réticulé dans l'appareil décrit ci-après. Elle permet en outre un contrôle en continu du degré de réticulation sur le produit en défilement en cours de fabrication et donc de la qualité des produits obtenus.

L'invention concerne donc un procédé de caractérisation par son taux de gel d'un échantillon gélifié, réticulé ou vulcanisé, caractérisé par les étapes suivantes :

a) On porte ledit échantillon à une température sensiblement constante et supérieure à sa température de fusion ou supérieure à sa température de transition vitreuse ;

b) On introduit ledit échantillon porté à ladite température dans un appareil de résonance magnétique nucléaire (RMN) ayant un rapport fréquence sur induction magnétique sensiblement constant et sensiblement égal à 42,5749 Megahertz · Tesla $^{-1}$ qui permet la résonance de l'hydrogène ou un rapport sensiblement constant et sensiblement égal à 40,0532 qui permet la résonance du fluor ;

c) On soumet ledit échantillon à au moins une séquence d'au moins deux impulsions de radiofréquence, la première à un angle Pi/2, la seconde à un angle Pi, avantageusement à au moins une séquence dite de Carr-Purcell, et de préférence à au moins une séquence d'au moins quatre impulsions ;

d) On recueille l'intensité du signal d'aimantation transversale ($M_x$) relatif à au moins un écho correspondant à un temps d'analyse compris entre 0,5 ms et 1 s, avantageusement compris entre 2 ms et 26 ms ;

e) Par comparaison avec une courbe d'étalonnage préétablie à l'aide de matériaux de référence de même composition, à taux de gel connus, selon les étapes a, b, c, et d ci-dessus et délivrant l'intensité des signaux d'aimantation transversale en fonction du taux de gel des matériaux de référence, on calcule à partir de l'intensité du signal de l'échantillon à caractériser ($M_x$) et de l'intensité des signaux des matériaux de référence au même dit écho, le taux de gel dudit échantillon.

On obtient ainsi très rapidement une méthode d'analyse fiable, précise reproductible et non destructive permettant de contrôler la qualité d'un produit et de modifier éventuellement de manière quasi simultanée les paramètres de fonctionnement et de régulation de la ligne de production.

On peut opérer en discontinu mais également en continu du fait d'un temps de mesure très court, par exemple de 5 à 10 secondes. Dans le cas où l'on opère en discontinu, on peut inverser les étapes a et b.

A la sortie de la ligne de fabrication, l'échantillon peut être au dessus de sa température de fusion ou de sa température de transition vitreuse. Dans ce cas, l'étape a) du procédé consiste en un maintien de la température à un niveau sensiblement constant.

L'échantillon peut aussi être à une basse température (20°C par exemple) à la sortie de la ligne de fabrication ; il faut alors le réchauffer selon l'étape a) du procédé afin de l'amener au dessus de la température de fusion ou de transition vitreuse dans le cas des élastomères ou des thermodurcissables.

Lorsqu'on opèrera en continu, l'échantillon sera effectivement à une température au dessus de sa température de fusion ou de transition vitreuse qu'il faudra maintenir.

On pourra faire défiler l'échantillon en continu dans l'aimant de l'appareil, dans son entrefer par exemple, à une vitesse comprise entre 0,01 et 40 m/min et de préférence entre 0,01 et 15 m/min.

Selon un autre mode de mise en oeuvre particulièrement avantageux du procédé, on pourra faire déplacer l'ensemble aimant et oscillateur de fréquence, ou l'oscillateur seul à une vitesse sensiblement égale à celle de l'échantillon qui défile dans l'aimant, pendant un temps au moins égal au temps d'analyse.

Selon un autre mode de réalisation du procédé, on peut améliorer la sensibilité de la méthode en procédant avant l'étape c) à un gonflement de l'échantillon avec un solvant organique dont la valeur du paramètre de solubilité est comprise entre celle du paramètre de solubilité de l'échantillon plus ou moins environ 4 et avantageusement plus ou moins 2. La quantité de solvant introduite est généralement dans un proportion comprise entre environ 0,01 et 10000% poids et avantageusement comprise entre 0,5 et 100% poids, ce qui permet d'obtenir une meilleure résolution du signal, et d'améliorer la sensibilité de la méthode. Il est bien entendu que le matériau de référence sera gonflé dans les mêmes conditions.

Parmi les solvants on peut citer par exemple les hydrocarbures aliphatiques et aromatiques tels que l'heptane, le toluène, les cétones telles que l'acétone, les éthers tels que l'éther diéthylique, les alcools, les esters, etc...

En règle générale, la valeur de l'induction magnétique est comprise entre 0,5 et 22 Tesla, avantageusement comprise entre environ 0,5 et 5 Tesla. La valeur des radiofréquences de l'oscillateur est généralement d'environ 15 à 1000 Megahertz et de préférence de 20 à 200 Megahertz. La température de l'échantillon introduit dans l'aimant est habituellement supérieure à sa température de fusion ou de transition vitreuse. Elle peut par exemple être supérieure d'environ 2 à 350°C à ces températures là.

Cet échantillon peut disposer de n'importe quelle section compatible avec le volume disponible soit dans l'entrefer de l'aimant dans le cas des aimants conventionnels, soit dans l'aimant dans le cas des aimants supra-conducteurs.

L'invention concerne aussi le dispositif qui peut également être utilisé en discontinu ou en continu (en ligne), de préférence, pour contrôler par exemple les paramètres de fabrication du produit. Il comporte un appareil de résonance magnétique nucléaire comprenant un aimant adapté à délivrer une induction magnétique comprise en général entre environ 0,5 et 22 Tesla et un oscillateur de radiofréquences adapté à délivrer une fréquence comprise entre environ 15 et 1000 Megahertz de telle sorte que le rapport fréquence sur induction soit sensiblement constant et connu pour un noyau d'atome donné choisi parmi l'hydrogène et le fluor, ledit appareil comprenant des moyens de positionnement d'un échantillon dans ledit aimant et étant adapté à délivrer au moins une séquence d'au moins deux impulsions, ledit appareil comportant en outre des moyens de détection des signaux d'aimantation transversale en fonction du temps, connectés à l'oscillateur de radiofréquence, ledit dispositif étant caractérisé en ce qu'il comprend :

a) des moyens de chauffage dudit échantillon à une température sensiblement constante reliés en général auxdits moyens de positionnement (porte-échantillon dans l'oscillateur de fréquence) et

b) des moyens de traitement desdits signaux connectés auxdits moyens de détection et adaptés à comparer le signal relatif dudit échantillon aux signaux relatifs desdits matériaux de référence et à calculer ledit taux de gel.

Lorsqu'on opère en continu, le dispositif peut comprendre des moyens de déplacement alternatif de l'ensemble aimant et oscillateur de fréquence ou de l'oscillateur seul adaptés à le déplacer à une vitesse sensiblement égale à celle dudit échantillon qui défile et reliés à des moyens de défilement de l'échantillon et des moyens d'asservissement connectés aux moyens de traitement des signaux délivrés par l'appareil de résonance magnétique et à des moyens de défilement, de régulation et de fonctionnement de la ligne de fabrication de l'échantillon produit.

Lorsque la vitesse de défilement est élevée (supérieure à 15 m/min par exemple), le dispositif peut comporter des seconds moyens d'asservissement reliés à des moyens de défilement de l'échantillon et auxdits moyens de déplacement alternatifs qui sont adaptés à asservir lesdits moyens de déplacement de l'ensemble aimant-oscillateur ou de l'oscillateur seul aux moyens de défilement dudit échantillon. Ces seconds moyens d'asservissement peuvent être reliés auxdits moyens de détection du signal comme on le verra ci-dessous.

L'invention concerne également le procédé d'utilisation en continu du dispositif dans lequel on contrôle le taux de gel d'un échantillon en matière plastique réticulée, gélifiée ou vulcanisée, et dans lequel on agit ensuite sur des moyens de fabrication de cet échantillon de manière à mener le taux de gel à la valeur désirée. Cela permet de maximiser la production du produit fabriqué. Par échantillon analysé en continu, on entend une partie du produit défilant dans l'appareil de RMN lors de l'analyse et qui n'est ni prélevée, ni détruite après l'analyse.

La méthode décrite utilise un spectromètre de résonance magnétique nucléaire réglé pour observer la résonance du noyau d'hydrogène ou du noyau de fluor dans le cas des polymères fluorés et équipé d'une sonde à température variable dans la gamme, de préférence, comprise entre 20 et 250°C

N'importe quel spectromètre commercial à impulsions peut convenir.

L'induction magnétique se situe en général entre 0,5 et 22 Tesla, la fréquence du courant dans la bobine excitatrice entre 15 et 1000 Megahertz avec impérativement un rapport fréquence sur induction magnétique égal à :

EP 0 313 435 B1

42,5749 ± 0,0005 Megahertz · Tesla⁻¹ pour la résonance de l'hydrogène,

40,0532 ± 0,0005 Megahertz · Tesla⁻¹ pour la résonance du fluor.

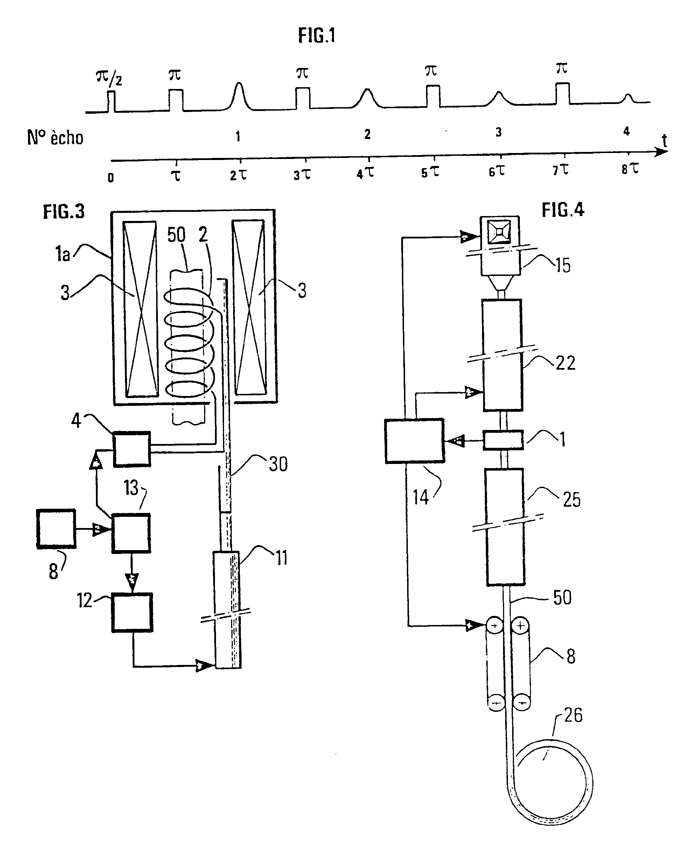

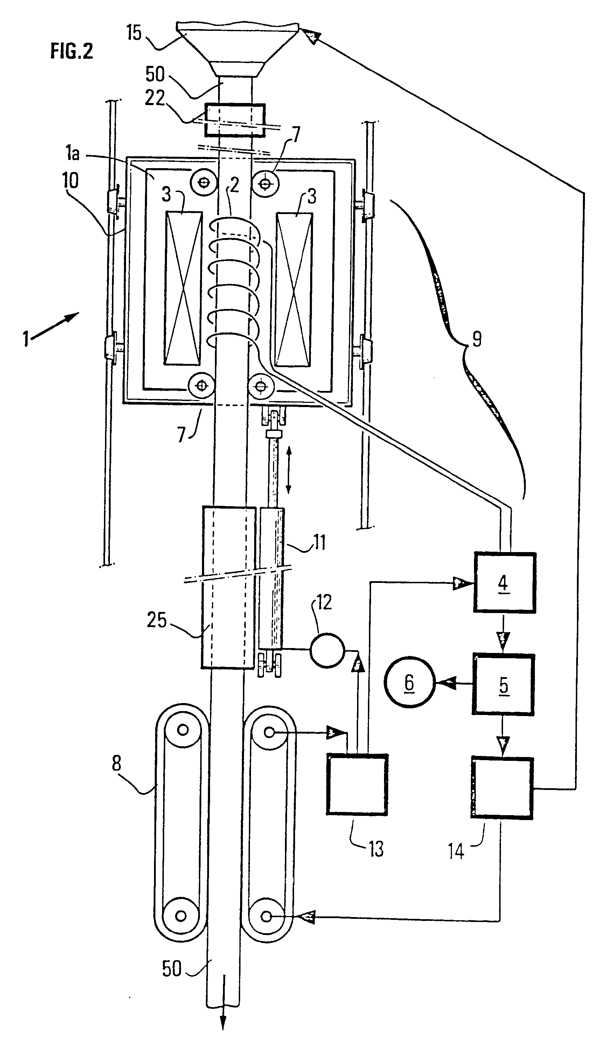

La caractérisation des échantillons se fait à partir de l'enregistrement de l'évolution de la relaxation de l'aimantation transversale en fonction du temps. Pour ce faire, on applique, en général, à l'échantillon placé dans l'induction magnétique, une impulsion de Pi/2 suivi après un temps $\tau$ pouvant varier de 0,1 à 20 ms d'une impulsion Pi. Un même temps $\tau$ après l'impulsion Pi, on recueille le signal de l'écho ainsi produit et on note son intensité maximum (séquence : Pi/2, $\tau$, Pi, $\tau$, Acquisition). Cette procédure est avantageusement remplacée par la séquence dite de Carr-Purcell qui consiste à appliquer au temps zéro une impulsion de Pi/2 puis à des temps $\tau$, 3 $\tau$, 5 $\tau$, etc... des impulsions Pi qui engendrent des échos aux temps 2 $\tau$, 4 $\tau$, 6$\tau$, etc... (voir figure 1), $\tau$ étant compris entre 0,1 et 2 ms. La variation de l'amplitude maximum de chacun des échos en fonction du temps constitue la fonction de relaxation de l'aimantation transversale.

Cette expérience est menée à une température supérieure à la température de transition vitreuse pour les élastomères et les thermodurcissables et supérieure au point de fusion pour les thermoplastiques.

La mesure du taux de gel peut se faire en comparant l'intensité maximum du $n^{\text{ième}}$ écho obtenu avec l'échantillon à mesurer avec le signal du même écho donné par un ou plusieurs échantillons dont le taux de gel est connu.

L'écho choisi est tel que les hydrogènes (ou les fluors) de la partie réticulée soient totalement relaxés ce qui amène à sélectionner des échos tels que le temps t soit habituellement supérieurs à 0,5 ms et inférieur à 30 ms (t, temps de mesure qui est égal à 2 $\tau$ si l'on emploie la séquence (Pi/2, $\tau$, Pi, $\tau$, Acquisition) et égal à 2 $n\tau$ si l'on emploie la séquence en Carr-Purcell, n étant alors le quantième de l'écho. On choisit préférentiellement t entre 8 et 12 ms.

De préférence, on utilise une séquence à 4 impulsions qui, elle, caractérise directement l'état réticulé, ce qui permet de s'affranchir d'effets parasites et de gagner en justesse. Cette séquence d'écho "pseudo solide" est constituée de la manière suivante :

pi/2, $\tau$, Pi, $\tau$, Pi/2, $\tau_1$, Pi,$\tau_1$, acquisition ; les deux impulsions
Pi/2 étant appliquées selon deux axes perpendiculaires Ox, Oy.

L'intensité du signal acquis après $\tau_1$ est retenue et comparée aux références.

La description, ci-dessus est relative à l'analyse des échantillons prélevés sur une production, le temps de retour de l'information est de l'ordre de quelques dizaines de secondes à quelques minutes et permet donc d'optimiser les conditions opératoires de fabrication.

Une autre variante de l'invention consiste à utiliser cette méthode de mesure en continu : le spectromètre est alors modifié de façon à permettre le passage en continu de l'échantillon dans l'induction magnétique. Dans ce cas, le dispositif fonctionne comme précédemment à condition que le temps de séjour de la partie excitée dans la zone d'irradiation soit supérieure au temps total de la mesure. Cette condition est généralement remplie si la vitesse de défilement de l'échantillon est inférieure à 15 m · min⁻¹. Si la vitesse de sortie de l'unité de fabrication est supérieure, on établit pendant le temps de mesure un déplacement de la bobine dans le même sens et à la même vitesse que l'échantillon sur un parcours de quelques centimètres (de préférence 1 cm) en s'appliquant à rester à induction constante. A la suite de la mesure, la bobine revient à son emplacement initial.

Dans le cas où la taille du profilé fabriqué est trop importante pour passer dans l'entrefer d'un électroaimant on peut utiliser un aimant supra-conducteur de diamètre suffisant qui permet en outre si l'échantillon contient une armature métallique interne non magnétique, d'utiliser des bobines de surface caractérisées par une faible pénétration des ondes électromagnétiques et permettant ainsi l'étude de l'objet réticulé seul.

L'invention sera mieux comprise au vu des figures et schémas ci-dessous montrant à titre purement illustratif un exemple de mise en oeuvre du procédé, parmi lesquels :

- la figure 1 montre la séquence d'impulsions soumise à l'échantillon à caractériser en fonction du temps;
- la figure 2 illustre le dispositif de contrôle et de caractérisation en routine d'un échantillon ;
- la figure 3 représente une variante du dispositif de contrôle en routine permettant d'asservir le déplacement de l'oscillateur de fréquence et
- la figure 4 montre un schéma d'une ligne de fabrication d'un profilé en caoutchouc réticulé comprenant le dispositif selon l'invention.

Selon la figure 2, le dispositif comprend un appareil 1 de résonance magnétique nucléaire du proton ou du fluor disposé sur un chassis 10, comportant un aimant 1a à l'intérieur duquel est disposé un oscillateur de radio-fréquence 2. Cet oscillateur fait office d'émetteur et de récepteur de signaux et est susceptible de loger un porte-échantillon contenant l'échantillon 50, si l'on travaille en discontinu, ou est susceptible de permettre le défilement de l'échantillon 50 en cours de fabrication, dans ses spires. Un moyen de chauffage 3 maintient l'échantillon ou le réchauffe à la température désirée grâce à des moyens de régulation de chauffage, connus en soi.

Les signaux reçus par l'oscillateur de fréquence 2 sont transmis à des moyens de détection 4, en fonction

5

du temps, puis ils sont traités par des moyens de traitement 5 où ils sont stockés et comparés à chaque instant souhaité à une courbe d'étalonnage établie sur des échantillons de même composition à taux de gel connus, dans les mêmes conditions opératoires. Un logiciel appliqué à ces moyens de traitement (microprocesseur) permet d'obtenir le résultat cherché, c'est-à-dire un taux de gel caractéristique de l'échantillon et éventuellement de le visualiser 6.

Il est alors possible de modifier éventuellement le paramètre de fonctionnement d'une unité de fabrication comme on le verra dans l'exemple ci-dessous.

Dans le cas d'un contrôle de fabrication en routine d'un profilé débouchant par exemple d'une extrudeuse 15 en amont du dispositif de mesure, le profilé défile à l'intérieur des spires de l'oscillateur 2 et est maintenu en traction par un moyen de défilement 8 qui peut être une chenille de tirage. Des moyens de positionnement 7 en amont et en aval du chassis 10, des guides ou des galets de centrage par exemple, non magnétiques de préférence, maintiennent le profilé en position centrée dans l'oscillateur. Des moyens d'asservissement 14 reliés aux moyens de traitement 5 des signaux permettent en général d'asservir des moyens de défilement 8 et d'extrusion 15 du profilé par comparaison du taux de gel souhaité que l'on affiche dans les moyens de traitement au taux de gel mesuré sur l'échantillon.

Des seconds moyens d'asservissement 13 peuvent être reliés aux moyens de défilement 8 du profilé et à des moyens de déplacement 9 du chassis 10 supportant l'appareil de résonance (aimant-bobine) de façon à asservir la vitesse de défilement de l'échantillon à la vitesse de déplacement du chassis. En effet, lorsque la vitesse de défilement de l'échantillon dépasse par exemple 15 m/min, on peut être amené à utiliser des moyens de déplacement 9 de l'ensemble aimant-oscillateur constitués par exemple d'un chassis non magnétique 10 positionné par exemple sur un rail et relié à un verin hydraulique ou pneumatique 11 connecté à un compresseur 12 assurant le déplacement alternatif du chassis à la fréquence correspondant à la vitesse de défilement de l'échantillon. Le déplacement conjoint de l'échantillon et du chassis supportant l'aimant et l'oscillateur de fréquence se fera pendant une période de temps légèrement supérieure au temps t de la mesure, par exemple 10 à 30 ms. Au moyen des seconds moyens d'asservissement de la vitesse de défilement de l'échantillon à la fréquence de déplacement alternatif du vérin et du chassis on déclenchera de préférence la séquence de mesure sur les moyens de détection 4 au début de la phase au cours de laquelle le chassis va dans le même sens et à une vitesse sensiblement identique à celle de l'échantillon.

Une variante de ce dispositif est illustré par la figure 3. Au lieu de déplacer le chassis 10 supportant l'aimant et l'oscillateur, on peut ne déplacer que l'oscillateur de fréquence à l'intérieur de l'aimant, par exemple dans son entrefer. Cet oscillateur est maintenu par un support 30 non magnétique qui est mis en mouvement par le vérin 11 relié au compresseur 12 qui est en relation avec les seconds moyens d'asservissement 13 qui asservissent, comme ci-dessus, la vitesse de défilement de l'échantillon à la vitesse de déplacement de l'oscillateur et à sa fréquence de déplacements alternatifs.

Les exemples suivants illustrent l'invention sans en limiter pour autant la portée.

## Exemple 1

Cette exemple concerne l'optimisation des conditions de fabrication de tube en polyéthylène réticulé utilisé dans l'industrie pétrolière pour convoyer du pétrole brut. La structure et la taille de ces tubes ne permettent généralement pas leur passage en continu dans l'entrefer d'un aimant de RMN. En effet, les diamètres sont variables suivant les fabrications de 2 pouces à 16 pouces et la présence d'un renfort métallique en inox à l'intérieur du tube perturberait trop le champ magnétique pour permettre une analyse correcte du taux de gel de la gaine interne d'étanchéité en polyéthylène réticulé. La structure de ces tubes est décrite dans le brevet FR-A-2.590.646. La composition de polyéthylène réticulé (PEhd) est décrite en détail dans le brevet FR-A-2.521.573. Un schéma de l'unité de fabrication de la gaine interne en PEhd réticulé est illustré par la figure 4.

Le feuillard agrafé en forme de tube, après avoir été préchauffé est amené dans une extrudeuse 15, de laquelle va déboucher la matière extrudée en forme de gaine. Ce tube gainé va être conduit dans un four de réticulation 22 (lit fluidisé) puis vers un bac de refroidissement au moyen d'une tireuse 8 qui l'envoie sur une bobine réceptrice d'enroulement.

Dans le cas d'un fonctionnement en discontinu, l'échantillon est prélevé après son passage dans le lit fluidisé à une température qui peut être au dessous de son point de fusion (par exemple vers 40°C). Sa taille est compatible avec celle du porte-échantillon que l'on introduit dans l'entrefer de l'aimant d'un appareil RMN type Minispec BRUCKER permettant la résonance du proton. La valeur de l'induction magnétique est de 0,46976 Tesla, la fréquence de l'oscillateur de radio-fréquence est de 20 Megahertz, le rapport fréquence sur induction magnétique étant de 42,5749 Megahertz · Tesla$^{-1}$. La température de l'échantillon est augmentée et maintenue constante à environ 145°C, température qui est supérieure à celle de son point de fusion (130°C). On déclenche la mesure : on applique une séquence d'impulsions de Carr-Purcell, c'est-à-dire une impulsion Pi/2 suivie à des temps 1, 3, 5, 7 et 9 ms d'impulsions Pi, et on recueille le signal écho M(x) au bout de 10 ms. Antérieurement, il a été déterminé une courbe d'étalonnage à partir d'échantillons de même matière à taux de gel connus, mesurés par la méthode ASTM D2756 et analysés dans les mêmes conditions opératoires suivant le procédé de l'invention ci-dessus (Tab. 1).

Tableau I

| % gel (ASTM D2756) | Signal RMN (145° C) |
|---|---|
| 0 | 0 |
| 15 | 0,15 |
| 47 | 0,30 |
| 77 | 0,41 |
| 86 | 0,55 |
| 91 | 0,68 |
| 94 | 0,80 |

Des essais de vieillissement préalable ont montré qu'un taux de gel supérieur à 75% était nécessaire pour assurer un bon comportement du produit lors de son utilisation.

Le tableau ci-dessous résume les diverses conditions de fabrication mises en oeuvre et l'optimisation qui a pu se faire rapidement grâce au système de mesure selon l'invention.

Tableau II

|  | CONDITIONS | | | | |
|---|---|---|---|---|---|
|  | n° 1 | n° 2 | n° 3 | n° 4 | n° 5 |
| Vitesse de vis d'extrudeuse (t/min) | 3 | 5 | 8 | 12 | 12 |
| Température pré-chauffage du feuillard | – | – | – | 180°C | 220°C |
| Température lit fluidisé | 260°C | 300°C | 330°C | 330°C | 330°C |
| Vitesse tirage (m/min) | 0,15 | 0,3 | 0,5 | 0,8 | 0,6 |
| Temps de séjour dans lit fluidisé (min) | 30 | 13 | 8 | 5 | 7 |
| Epaisseur de gaine (mm) en polyéthylène réticulé | 7 | 7 | 7 | 7 | 7 |
| Taux de gel mesuré selon l'invention (%) | 78 | 91 | 91 | 68 | 91 |

L'intérêt de la méthode réside principalement dans la rapidité de la mesure qui permet d'obtenir en quelques minutes un résultat fiable permettant d'agir sur la modification des paramètres de fonctionnement de l'installation avant que la production n'ait réellement commencé (condition n° 4) et d'optimiser la vitesse de fabrication de l'unité de production de flexibles (condition n° 5). Pour vérifier les résultats obtenus, une analyse selon la norme ASTM D2756 a été réalisée à posteriori sur les échantillons ayant servi à la mesure du taux de gel par RMN (la méthode étant non destructive). Les résultats obtenus après plusieurs jours d'extraction et de séchage figurent au tableau III et illustrent clairement la fiabilité de la nouvelle méthode proposée.

Tableau III

| | CONDITIONS N° | | | | |
|---|---|---|---|---|---|
| | n° 1 | n° 2 | n° 3 | n° 4 | n° 5 |
| Taux de gel mesuré par RMN | 78 | 91 | 91 | 68 | 91 |
| Taux de gel selon ASTM D2756 | 79 | 90 | 91 | 66 | 91 |

## Exemple 2

On considère dans l'exemple suivant le cas où l'on contrôle en continu la ligne de fabrication d'un profilé en caoutchouc de section 2 cm² qui défile dans l'oscillateur de fréquence à une vitesse de défilement inférieure à 15 m/min. L'appareil 1 selon l'invention a été placé entre le four de réticulation 22 et le bac de refroidissement 25. La vitesse de défilement de l'échantillon est de 5 m/min. La température de l'échantillon est d'environ 200°C et la température de mesure selon l'invention est d'environ 198°C, toutes les autres conditions de la mesure étant les mêmes que celles de l'exemple 1 et la courbe d'étalonnage ayant été établie préalablement à 198°C avec des matériaux de même composition.

On analyse le profilé toutes les 10 secondes.

L'information recueillie est analysée dans le microordinateur 4 et sert à asservir la vitesse de vis de l'extrudeuse et la vitesse de tirage du profilé de manière à maintenir constante à la fois la section du profilé et le taux de gel du produit fabriqué. La composition étudiée est la suivante :

```
Caoutchouc EPDM   :      100 phr    Vistalon 2504 de chez ESSO

                         phr (per hundred rubber)

Perkadox BC              1,5 phr

(péroxyde de dicumyl)
```

L'étude des conditions de vulcanisation au vulcamètre montre qu'un temps de séjour de 3 minutes à 200°C permet d'obtenir un taux de gel satisfaisant de 93%, un taux de gel supérieur à 85% étant suffisant pour atteindre les caractéristiques requises par le profilé.

Au départ de la fabrication, l'unité de fabrication a été réglée de manière à ce que le temps de séjour du profilé dans le lit fluidisé de réticulation à 200°C soit de 6 min, la vitesse linéaire du profilé étant dans ce cas de 2,5 m/min.

Les informations fournies par le dispositif de mesure montrent que le taux de gel mesuré selon l'invention dans ce cas est de l'ordre de 94%.

On agit sur les paramètres de contrôle de la ligne par les moyens d'asservissement. La vitesse linéaire de défilement du profilé est alors augmentée pour atteindre 5 m/min et, après stabilisation du fonctionnement de l'unité un taux de gel constant de 92% à été mesuré pendant plusieurs heures de fabrication.

## Exemple 3

On considère dans cet exemple le cas où la vitesse de défilement du profilé qui passe dans l'appareil RMN est supérieure à 15 m/min. Le dispositif selon l'invention peut être disposé par exemple entre la tireuse 8 et la bobine réceptrice 26 et non plus (comme indiqué sur la figure 4) entre le four 22 et le bac de refroidissement 25.

La gaine est constituée du même matériau que celui de l'exemple 2. Elle est produite à une vitesse de 40 m/min. La température du four de réticulation 22 est de 220°C. On refroidit la gaine dans le bac de refroidissement 25 et la température tombe aux environs de 40°C. On effectue le procédé de caractérisation du profilé selon l'invention dans les mêmes conditions que celles de l'exemple précédent, à l'exception de la température de RMN du proton qui est maintenue à 40°C, la courbe d'étalonnage ayant été préétablie à cette même température. La température de transition vitreuse de l'échantillon est de −20°C.

On asservit le déplacement alternatif du chassis à la vitesse de défilement de la gaine soit 1 cm en 15 ms dans la direction de défilement, grâce aux seconds moyens 13 d'asservissement (type Eurotherm [R]) reliés au compresseur 12 et au vérin 11 agissant sur le chassis 10. Le chassis est ensuite ramené à son point de départ et on déclenche une séquence de mesures selon l'invention, par exemple toutes les 30 ms ou tous les multiples de 30 ms. On obtient dans ces conditions un taux de gel de 85% qui n'est pas satisfaisant. On régule alors grâce aux moyens 14 d'asservissement (type Eurotherm [R]) la température du four de réticulation 22 à 240°C et après une mise en équilibre de la ligne de production, on constate que le taux de gel atteint progressivement 93%.

Selon la variante décrite, particulièrement avantageuse en raison de sa simplicité de mise en oeuvre, on pourrait déplacer légèrement l'oscillateur de fréquence à la vitesse ci-dessus, au lieu de déplacer le chassis supportant l'ensemble aimant-oscillateur.

Le dispositif selon l'invention a donc permis dans ces cas d'exemples d'optimiser les conditions de fabrication et d'améliorer le rendement de l'installation. De plus le dispositif d'asservissement 14 permet d'éviter toute dérive de fonctionnement de l'unité et de garantir les caractéristiques du produit fabriqué.

## Exemple 4

On reprend l'exemple 2 mais au lieu d'utiliser une séquence d'impulsions de Carr-Purcell, on utilise une séquence à 4 impulsions dite méthode de l'écho pseudo-solide obtenue par exemple par l'appareil BRUCKER type CXP. Ces trains d'impulsions de radiofréquence ont différentes phases x, −x, y, −y avec x et y déphasé de 90°C.

L'échantillon défilant est soumis à la séquence d'impulsions suivante :

$$(\frac{Pi}{2})y, \ \tau, \ (Pi)_y, \ \tau, \ (\frac{Pi}{2}) - x, \ \tau_1, \ (Pi)_y, \ \tau_1$$

avec un temps $\tau$ égal à 2,5 ms et $\tau_1$ égal à 0,5 ms.

Le signal acquis 0,5 ms après la dernière impulsion permet une mesure plus juste car directement proportionnelle au taux de réticulation. La valeur du signal est comparée à celles correspondant à des échantillons de référence obtenues dans les mêmes conditions opératoires.

## Revendications

1. Procédé de fabrication d'un produit gélifié réticulé ou vulcanisé ayant un taux de gel à une valeur désirée, comportant une ligne de fabrication en continu dudit produit, caractérisé en ce que

1/ on contrôle en continu ledit taux de gel d'un échantillon dudit produit par les étapes successives suivantes :

a) on maintient ledit échantillon à une température sensiblement constante et supérieure à sa température de fusion ou supérieure à sa température de transition vitreuse ;

b) on fait défiler en continu ledit échantillon porté à ladite température dans un appareil de résonance magnétique nucléaire (RMN) comportant un aimant et un oscillateur de fréquence et ayant un rapport fréquence sur induction magnétique sensiblement constant et sensiblement égal à 42,5749 Megahertz · Tesla$^{-1}$ qui permet la résonance de l'hydrogène ou un rapport sensiblement constant et sensiblement égal à 40,0532 Megahertz · Tesla$^{-1}$ qui permet la résonance du fluor ;

c) on soumet ledit échantillon à au moins une séquence d'au moins deux impulsions de radiofréquence, la première à un angle Pi/2, la seconde à un angle Pi, avantageusement à au moins une séquence dite de Carr-Purcell, et de préférence à au moins une séquence d'au moins quatre impulsions ;

d) on recueille l'intensité du signal d'aimantation transversale ($M_x$) relatif à au moins un écho correspondant à un temps d'analyse compris entre 0,5 ms et 1 s, avantageusement compris entre 2 ms et 26 ms ;

e) par comparaison avec une courbe d'étalonnage préétablie à l'aide de matériaux de référence de même composition, à taux de gel connus, selon les étapes a, b, c et d ci-dessus et délivrant l'intensité des signaux d'aimantation transversale en fonction du taux de gel des matériaux de référence, on calcule à partir de l'intensité du signal de l'échantillon à caractériser ($M_x$) et de l'intensité des signaux des matériaux de référence au même dit écho, le taux de gel dudit échantillon, et

2/ on agit sur la ligne de fabrication dudit produit de manière à mener le taux de gel à la valeur désirée.

2. Procédé selon la revendication 1 dans lequel on fait défiler ledit échantillon en continu dans l'appareil de résonance magnétique nucléaire à une vitesse comprise entre 0,01 et 40 m/min et de préférence entre 0,01 et 15 m/min.

3. Procédé selon les revendications 1 et 2 dans lequel on fait déplacer ledit appareil à une vitesse sensiblement égale à celle dudit échantillon pendant un temps au moins égal audit temps d'analyse.

4. Procédé selon la revendication 1 dans lequel on fait déplacer uniquement ledit oscillateur à une vitesse sensiblement égale à celle de l'échantillon pendant un temps au moins égal au temps d'analyse.

5. Procédé selon l'une des revendications 1 à 4 dans lequel ladite induction magnétique est comprise entre environ 0,5 et 22 Tesla, avantageusement comprise entre environ 0,5 et 5 Tesla et la fréquence est comprise entre 15 et 1000 Megahertz et avantageusement entre 20 et 200 Megahertz.

6. Procédé selon l'une des revendications 1 à 5 dans lequel la température dudit échantillon est maintenue constante et est supérieure d'environ 2 à 350°C à sa température de fusion ou à sa température de transition vitreuse.

7. Dispositif pour la mise en oeuvre du procédé selon l'une des revendications 1 à 6 comprenant des moyens (8, 15, 22) de défilement, de régulation et de fonctionnement d'une ligne de fabrication dudit produit, caractérisé en ce qu'il comprend

a) un appareil (1) de résonance magnétique nucléaire comprenant un aimant (1a) adapté à délivrer une induction magnétique comprise entre environ 0,5 et 22 Tesla et un oscillateur de fréquence (2) adapté à délivrer une fréquence comprise entre environ 15 et 1000 Megahertz, de telle sorte que le rapport fréquence sur induction magnétique soit sensiblement constant et connu pour un noyau d'atome donné choisi parmi l'hydrogène et le fluor, ledit appareil étant adapté à délivrer au moins une séquence d'au moins deux impulsions, ledit appareil comportant en outre des moyens de détection (4) des signaux d'aimantation transversale en fonction du temps connectés à l'oscillateur de fréquence (2),

b) des moyens de positionnement (7) dudit échantillon de produit adaptés à positionner l'échantillon durant son défilement en continu dans l'aimant et l'oscillateur,

c) des moyens de chauffage (3) dudit échantillon adaptés à le maintenir à une température sensiblement constante durant le défilement,

d) des moyens de traitement (5) desdits signaux connectés auxdits moyens de détection (4) et adaptés à comparer le signal relatif audit échantillon aux signaux relatifs auxdits matériaux de référence et à calculer ledit taux de gel, et

e) des moyens d'asservissement (14) connectés auxdits moyens de traitement (5) et aux moyens (8, 15, 22) de défilement, de régulation et de fonctionnement de la ligne de fabrication dudit échantillon.

8. Dispositif selon la revendication 7 caractérisé en ce qu'il comprend des moyens de déplacement (9) alternatif de l'ensemble aimant et oscillateur de fréquence ou de l'oscillateur seul, adaptés à le déplacer à une vitesse sensiblement égale à celle dudit échantillon qui défile et reliés aux moyens (8) de défilement de l'échantillon.

9. Dispositif selon les revendications 7 et 8 caractérisé en ce qu'il comporte des seconds moyens d'asservissement (13) reliés auxdits moyens (8) de défilement de l'échantillon et auxdits moyens (9) de déplacement alternatif de l'aimant, qui sont adaptés à asservir lesdits moyens de déplacement de l'ensemble aimant-oscillateur ou de l'oscillateur seul auxdits moyens de défilement dudit échantillon et en ce que lesdits seconds moyens d'asservissement sont reliés auxdits moyens de détection du signal.

10. Dispositif selon l'une des revendications 7 à 9 dans lequel l'aimant est un aimant supra-conducteur.

## Ansprüche

1. Verfahren zur Herstellung eines gelierten vernetzten oder vulkanisierten Produkts, das einen Gelanteil von einem gewünschten Wert hat, mit einer kontinuierlichen Produktionsstraße für dieses Produkt, dadurch gekennzeichnet, daß

1/ man kontinuierlich diesen Gelanteil einer Probe dieses Produkts nach den folgenden Schritten regelt :

a) man hält diese Probe auf einer im wesentlichen konstanten Temperatur, die höher als ihre Schmelztemperatur oder höher als ihre Glasumwandlungstemperatur ist ;

b) man läßt kontinuierlich diese auf diese Temperatur gebrachte Probe in einer Kernspinnresonanzvorrichtung (RMN) durchlaufen, die einen Magneten und einen Frequenzoszillator umfaßt und ein im wesentlichen konstantes Verhältnis von Frequenz zu magnetischer Induktion und im wesentlichen gleich 42,5749 Megahertz · Tesla$^{-1}$, was die Resonanz des Wasserstoffs erlaubt, hat, oder ein im wesentlichen konstantes Verhältnis, welches im wesentlichen gleich 40,0532 Megahertz · Tesla $^{-1}$, was die Resonanz des Fluors ermöglicht, hat ;

c) man setzt diese Probe wenigstens einer Sequenz wenigstens zweier Radiofrequenzimpulse, die erste bei einem Winkel Pi/2, die zweite bei einem Winkel Pi, vorzugsweise wenigstens gleich einer sogenannten Carr-Purcell-Sequenz ist, und vorteilhaft wenigstens einer Sequenz von wenigstens vier Impulsen aus ;

d) man sammelt die Stärke des Quermagnetisierungssignals ($M_x$) relativ zu wenigstens einem Echo entsprechend einer Analysenzeit zwischen 0,5 ms und 1 s, vorzugsweise zwischen 2 ms und 26 ms ;

e) durch Vergleich mit einer vorher mit Hilfe von Bezugsmaterialien gleicher Zusammensetzung aufgestellten Eichkurve bei bekannten Gelanteilen entsprechend den obigen Stufen a, b, c und d und die die Stärke der Quermagnetisierungssignale als Funktion des Gelanteils der Bezugsmaterialien liefert, berechnet man ausgehend von der Stärke des zu charakterisierenden Probesignals ($M_x$) und der Stärke der Signale der Bezugsmaterialien bei diesem gleichen Echo den Gelanteil dieser Probe und

2/ man wirkt auf die Produktionsstraße für dieses Produkt derart ein, daß der Gelanteil auf den gewünschten Wert geführt wird.

2. Verfahren nach Anspruch 1, bei dem man diese Probe kontinuierlich in der Kernspinnresonanzvorrichtung bei einer Geschwindigkeit zwischen 0,01 und 40 m/Min. und vorteilhaft zwischen 0,01 und 15 m/Min. durchlaufen läßt.

3. Verfahren nach den Ansprüchen 1 und 2, bei dem man die Bewegung dieser Vorrichtung bei einer Geschwindigkeit im wesentlichen gleich der dieser Probe während eines Zeitraums wenigstens gleich diesem Analysenzeitraum veranlaßt.

4. Verfahren nach Anspruch 1, bei dem man die Bewegung allein dieses Oszillators bei einer Geschwindigkeit im wesentlichen gleich der der Probe während eines Zeitraums wenigstens gleich dem Analysenzeitraum veranlaßt.

5. Verfahren nach einem der Ansprüche 1 bis 4, bei dem diese magnetische Induktion zwischen etwa 0,5 und 22 Tesla, vorzugsweise etwa zwischen 0,5 und 5 Tesla beträgt und die Frequenz zwischen 15 und 1000 Megahertz und vorzugsweise zwischen 20 und 200 Megahertz liegt.

6. Verfahren nach einem der Ansprüche 1 bis 5, bei dem die Temperatur dieser Probe konstant und höher als etwa 2 bis 350°C als ihre Schmelztemperatur oder ihre Glasumwandlungstemperatur gehalten wird.

7. Vorrichtung zur Durchführung des Verfahrens nach einem der Ansprüche 1 bis 6 mit Mitteln (8, 15, 22) zum Durchlauf, Steuern und Betreiben einer Fabrikationsstraße für dieses Produkt, dadurch gekennzeichnet, daß sie umfaßt

a) eine kernmagnetische Resonanzvorrichtung (1) mit einem Magnet (1a), der so ausgelegt ist, daß er eine magnetische Induktion zwischen etwa 0,5 und 22 Tesla liefert und einen Frequenzoszillator (2) derartiger Auslegung, daß er eine Frequenz zwischen etwa 15 und 1000 Megahertz derart liefert, daß das Verhältnis Frequenz zu magnetischer Induktion im wesentlichen konstant und für einen gegebenen Atomkern bekannt ist, der aus Wasserstoff und Fluor gewählt ist, wobei diese Vorrichtung so ausgelegt ist, daß sie wenigstens eine Sequenz wenigstens zweier Impulse liefert, wobei die Vorrichtung im übrigen Mittel (4) zum Detektieren der Transversalmagnetisierungssignale als Funktion der Zeit umfaßt, die mit dem Frequenzoszillator (2) verbunden sind,

b) Mittel zum Positionieren (7) dieser Produktprobe derartiger Auslegung, daß die Probe während ihres Durchlaufs kontinuierlich im Magneten und dem Oszillator positioniert wird,

c) Mittel zum Heizen (3) dieser Probe derartiger Auslegung, daß sie auf einer im wesentlichen konstanten Temperatur während des Durchlaufs gehalten wird,

d) Mittel zur Verarbeitung (5) dieser Signale, die mit den Detektoreinrichtungen (4) verbunden und so ausgelegt sind, daß sie das sich auf diese Probe beziehende Signal mit den auf diese Bezugsmaterialien sich beziehenden Signalen vergleichen und diesen Gelanteil berechnen, und

e) Steuermittel (14), die mit den Verarbeitungsmitteln (5) und den Mitteln (8, 15, 22) zum Durchlauf, Steuern und Betrieb der Fabrikationsstraße dieser Probe verbunden sind.

8. Vorrichtung nach Anspruch 7, dadurch gekennzeichnet, daß sie Mittel zur alternativen Bewegung (9) der aus Magnet und Frequenzoszillator oder Oszillator allein bestehenden Anordnung umfaßt und die so ausgelegt sind, daß sie sie bei einer Geschwindigkeit im wesentlichen gleich der dieser Probe, die durchläuft, bewegt oder verschiebt und wobei diese mit den Durchlaufmitteln (8) der Probe verbunden sind.

9. Vorrichtung nach den Ansprüchen 7 und 8, dadurch gekennzeichnet, daß sie zweite Steuermittel (13) umfaßt, die mit diesen Mitteln (8) zum Durchlauf der Probe und mit den Mitteln (9) zur alternativen Bewegung des Magneten verbunden sind, die so ausgelegt sind, daß sie diese Bewegungsmittel der Magnet-Oszillatoranordnung oder der Oszillatoranordnung allein bezüglich der Durchlaufmittel diese Probe steuern und daß diese zweiten Steuermittel mit diesen Signaldetektormitteln verbunden sind.

10. Vorrichtung nach einem der Ansprüche 7 bis 9, bei der der Magnet ein supra-leitender Magnet ist.

## Claims

1. A method of producing a cross-linked or vulcanised gellified product having a gelling rate at a desired value, comprising line for the continuous manufacture of the said product, characterised in that

1/ the said gelling rate of a sample of the said product is continuously monitored by the following successive stages :

a) the said sample is maintained at a substantially constant temperature which is greater than its melting temperature or greater than its vitreous transition temperature ;

b) the said sample, raised to the said temperature, is passed continuously through a nuclear magnetic resonance (NMR) apparatus comprising a magnet and a frequency oscillator and having a ratio of frequency to magnetic induction which is substantially constant and substantially equal to 42.5749 Megahertz · Tesla$^{-1}$ which permits resonance of the hydrogen or a substantially constant ratio which is substantially equal to 40.0532 Megahertz · Tesla$^{-1}$ which allows fluorine resonance ;

c) the said sample is subjected to at least one sequence of at least two radiofrequency pulses, the first at an angle Pi/2, the second at an angle Pi advantageously to at least one so-called Carr-Purcell sequence and preferably to at least one sequence of at least four pulses ;

d) the intensity of the transverse magnetisation signal (Mx) relative to at least one echo corresponding to an analysis time comprised between 0.5 ms and 1 s and advantageously comprised between 2 ms and 26 ms is acquired ;

e) by comparison with a calibration curve pre-established by means of reference materials of the same composition, of known gelling rates, according to stages a, b, c and d above and furnishing the intensity of transverse magnetising signals as a function of the gelling rates of the reference materials, the gelling rate of the said sample is calculated on a basis of the intensity of the signal of the sample to be characterised (Mx) and the intensity of the signals of the reference materials at the same said echo ; and

2/ the line manufacturing the said product is acted upon in such a way as to bring the gelling rate to the desired value.

2. A method according to Claim 1 in which the said sample is passed continuously through the nuclear magnetic resonance apparatus at a speed comprised between 0.01 and 40 m/min and preferably between 0.01 and 15 m/min.

3. A method according to Claims 1 and 2 in which the said apparatus is moved at a speed substantially equal to that of the said sample for a period which is at least equal to the said analysis time.

4. A method according to Claim 1 in which only the said oscillator is moved at a speed substantially equal to that of the sample for a time which is at least equal to the analysis time.

5. A method according to one of Claims 1 to 4, in which the said magnetic induction is comprised between approx. 0,5 and 22 Tesla, advantageously comprised between approx. 0.5 and 5 Tesla and the frequency is comprised between 15 and 1000 Megahertz and is advantageously between 20 and 200 Megahertz.

6. A method according to one of Claims 1 to 5 in which the temperature of the said sample is maintained constant and is greater than its melting temperature or its vitreous transition temperature by approx. 2 to 350°C.

7. An apparatus for carrying out the method according to one of Claims 1 to 6, comprising means (8, 15, 22) of displacing, regulating and operating a line for producing the said product, characterised in that it comprises

a) a nuclear magnetic resonance apparatus (1) comprising a magnet (1a) adapted to deliver magnetic induction of between approx. 0.5 and 22 Tesla and a frequency oscillator (2) adapted to deliver a frequency comprised between approx. 15 and 1000 Megahertz, so that the frequency : magnetic induction ratio is substantially constant and known for a given atomic nucleus chosen between hydrogen and fluorine, the said apparatus being adapted to deliver at least one sequence of at least two pulses, the said apparatus further comprising means (4) for detecting transverse magnetisation signals as a function of the connection time to the frequency oscillator (2),

b) means (7) of positioning the said product sample adapted to position the sample while it is passing continuously through the magnet and the oscillator,

c) means (3) of heating the said sample and adapted to maintain it at a substantially constant temperature during passage,

d) means (5) of processing the said signals connected to the said detection means (4) and adapted to compare the signal relating to the said sample with the signals relating to the said reference materials and adapted to calculate the said gelling rate and

e) automatic control means (14) connected to the said processing means (5) and the said means (8, 15, 22) of displacement, regulation and operation of the line producing the said sample.

8. An apparatus according to Claim 7, characterised in that it comprises alternating means (9) of displacement of the magnet and frequency oscillator assembly or of the oscillator by itself, adapted to displace it at a speed substantially equal to that of the said sample which is passing and connected to the sample displacement means (8).

9. An apparatus according to Claims 7 and 8, characterised in that it comprises second automatic control means (13) connected to the said sample displacement means (8) and the said means (9) for alternating displacement of the magnet, which are adapted to control the said means of displacing the magnet-oscillator assembly or the oscillator by itself subject to the said sample displacement means and in that the said second control means are connected to the said signal detection means.

10. An apparatus according to one of Claims 7 to 9 in which the magnet is a supra-conductor magnet.

## FIG.1

π/2    π         π         π         π

N° ècho        1         2         3         4        t

0    τ    2τ    3τ    4τ    5τ    6τ    7τ    8τ

## FIG.3

1a
3
50  2
3
4
13
8
12
30
11

## FIG.4

15
22
1
14
25
50
8
26

**FIG.2**